(19) Europäisches Patentamt
European Patent Office
Office européen des brevets

(11) Numéro de publication: **0 027 421**
**B1**

# (12) FASCICULE DE BREVET EUROPEEN

(45) Date de publication du fascicule du brevet: **29.12.82**

(21) Numéro de dépôt: **80401457.9**

(22) Date de dépôt: **10.10.80**

(51) Int. Cl.³: **C 07 C 147/08,** C 07 C 147/04, B 01 J 23/44

(54) **Procédé de préparation de sulfones insaturées et sulfones obtenues.**

(30) Priorité: **11.10.79 FR 7925319**

(43) Date de publication de la demande: **22.04.81 Bulletin 81/16**

(45) Mention de la délivrance du brevet: **29.12.82 Bulletin 82/52**

(84) Etats contractants désignés: **AT BE CH DE GB IT LI LU NL SE**

(56) Documents cités:
**FR-A-1 425 317**
**US-A-3 674 856**
**US-A-3 711 516**

(73) Titulaire: **ANVAR Agence Nationale de Valorisation de la Recherche, 43, rue Caumartin, F-75436 Paris Cedex 09 (FR)**

(72) Inventeur: **Julia, Marc, 57 rue Geoffroy St. Hilaire, F-75005 Paris (FR)**
Inventeur: **Saussine, Lucien, 18, Avenue Villemain, F-75014 Paris (FR)**

(74) Mandataire: **Corre, Jacques Denis Paul et al, Cabinet Regimbeau 26, Avenue Kléber, F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

Procédé de préparation de sulfones insaturées et sulfones obtenues

La présente invention concerne un procédé de préparation de sulfones insaturées, en particulier de sulfones allyliques.

L'utilité en synthèse des sulfones, en particulier des sulfones allyliques comme cela est décrit dans P. D. Magnus, Tetrahedron (Report), 1977, 33, 2019, notamment comme intermédiaire de synthèse dans l'élaboration de molécules importantes comme les terpènes et les vitamines A et E, conduit à rechercher de nouvelles voies d'accès à de telles sulfones.

Il est connu de préparer de telles sulfones allyliques par action d'alcool allylique en milieu acide ou d'halogénure allylique sur des sulfinates alcalins. Les halogénures allyliques sont souvent obtenus eux-mêmes à partir des diènes conjugués. Ce type de synthèse est utilisé, en particulier, pour la préparation de la prénylsulfone et de la géranylsulfone qui sont des intermédiaires importants pour accéder, respectivement, à l'acide chrysanthémique, aux ionones et aux vitamines A et E.

Toutefois, la préparation, la stabilité et la réactivité des halogénures allyliques laissent à désirer et présentent de nombreux inconvénients sur le plan pratique. En outre, les halogénures allyliques étant eux-mêmes préparés à partir des diènes correspondants, il était intéressant de trouver un procédé permettant de préparer directement les sulfones allyliques à partir des diènes.

C'est pourquoi, la présente invention concerne un procédé de préparation de sulfones de formule I:

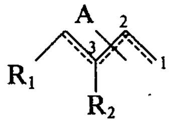

(I)

dans laquelle, la ligne pointillee représente une double liaison en position 1, 2 ou 3 et A représente un radical alkylsulfonyle, alkylensulfonyle ou arylsulfonyle, placé en $\alpha$ de ladite double liaison, $R_1$ et $R_2$ représentent l'hydrogène, un radical alkyle, alkényle ou alkynyle substitués ou bien substitués par un ou plusieursfonctions hydroxy ou oxo et/ou par des radicaux cycloalkyles ou cycloalkényles qui peubert eux-mêmes être substitués par un ou plusieurs radicaux methyles, par sulfonation, en présence d'un catalyseur au palladium, d'un composé diénique de formule II

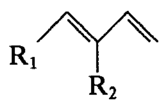

(II)

dans laquelle $R_1$ et $R_2$ ont les significations données précédemment, à l'aide d'au moins un agent introduisant le radical sulfonyle substitué A choisi parmi:

- les acides sulfiniques,
- les sulfinates en présence d'un acide,

dans un solvant, et séparation des sulfones et isomerisation de certaines d'entre elles, si cela est nécessaire.

Dans le cadre de la présente description, il faut entendre par »radical alkyle«, de préférence des radicaus alkyles droits ou ramifiés ayant de 1 à 30 atomes de carbone et de préférence de 1 à 10 atomes de carbone tels que les radicaux méthyle, éthyle ou propyle.

De même, les radicaux alkényles osnt, de préférence, des radicaux alkényles droits ou ramifiés, ayant de 2 à 30 atomes de carbone et de préférence de 2 à 10 atomes de carbone, lesdits radicaux pouvant présenter plusieurs doubles liaisons de preférence conjuguées, tels que les radicaux éthylényle ou méthyl-4 pentèn-3 yle.

Enfin, les radicaux alkynyles sont, de préférence, des radicaux alkynyles droits ou ramifiés, ayant de 2 à 30 atomes de carbone et de préférence de 2 à 10 atomes de carbone.

Ces différents radicaux peuvent également être substitués par une ou plusieurs fonctions hydroxy ou oxo et/ou par des radicaux cycloalkyles ou cycloalkényles, de préférence à 6 atomes de carbone dans le cycle, et qui peuvent eux-mêmes être substitués par un ou plusieurs radicaux méthyles.

Parmi les radicaux arylsulfonyles, il faut citer plus particulièrement les radicaux phénylsulfonyles non substitués ou substitués par un ou plusieurs radicaux alkyles inférieurs, en particulier méthyle, tels que les radicaux toluènesulfonyles.

Parmi les composés diéniques de formule II particulièrement intéressants pour la mise en oeuvre du procédé selon la présente invention, il faut citer le butadiène, l'isoprène, le myrcène et le pipérylène.

Parmi les composés introduisant le radical arylsulfonyle A, il faut citer plus particulièrement les acides arylsulfiniques tels que l'acide phénylsulfinique ou l'acide p-toluènesulfinique, le mélange d'acide arylsulfinique et d'un sulfinate correspondant ou bien l'utilisation d'un arylsulfinate en présence d'un acide faible tel que l'acide acétique ou l'acide formique. Par commodité, on utilisera de

préférence les arylsulfinates alcalins.

Parmi les catalyseurs au palladium, il faut citer plus particulièrement l'utilisation conjointe d'un sel ou d'un complexe de palladium tel qu'un dérivé $\pi$-allyl-palladié et d'une phosphine, en particulier l'utilisation de bis-$\pi$-allylchloropalladium et de triphénylphosphine, mais il est possible également d'utiliser d'autres complexes de palladium tels que le tétrakis-triphénylphosphine palladium (0) en presence d'une triphénylphosphine.

La réaction est conduite, de préférence, dans un solvant organique tel que le tétrahydrofuranne (THF).

Comme on préfère conduire la réaction à l'état liquide, et que la majorité des diènes utilisés sont des gaz, on opérera de préférence en récipient clos à des températures comprises entre environ 5° C et environ 100° C, de préférence entre 20 et 80° C. La réaction peut être conduite sous atmosphère inerte, argon par exemple.

Bien qu'il soit possible d'utiliser des quantités équimolaires de diène et d'agent introduisant le radical A, on préfère utiliser un excès de diène pouvant aller jusqu'à 20/1 en moles.

La quantité de catalyseur utilisée n'est pas un paramètre critique, elle peut varier dans de larges limites.

Toutefois, le rapport molaire entre le dérivé de palladium et la phosphine devra, en général, être d'au moins 1 et pourra atteindre 1/10 ou plus.

Après quelques dizaines d'heures de réaction, on obtient, en général, un mélange de sulfones de formule I avec, en général, comme produit principal le dérivé sulfonique le plus substitué.

Les différentes sulfones du mélange peuvent être séparées par tous procédés connus, en particulier il est possible d'effectuer des recristallisations dans des mélanges éthérés et/ou des séparations par chromatographie ou simple distillation.

Comme les sulfones particulièrement intéressantes sont les sulfones primaires, il est possible d'isomériser les sulfones de formule Ia:

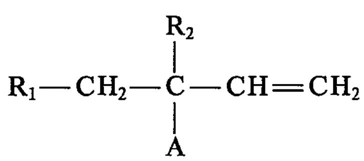

(Ia)

afin de préparer des sulfones de formule Ib:

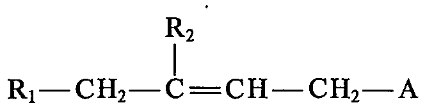

(Ib)

par action d'un sulfinate, par exemple un sulfinate alcalin, de préférence en présence d'un solvant organique tel que le THF et d'eau, à une température comprise entre 5° C et la température de reflux du mélange.

Le sulfinate utilisé est, de préférence, le sulfinate correspondant au radical A.

Dans ces conditions, on obtient, avec un rendement pratiquement quantitatif, les sulfones de formule Ib éventuellement sous forme d'un mélange des isomères E et Z.

Il est possible, si on le désire, d'effectuer l'isomérisation des sulfones directement dans le mélange réactionnel de l'étape de synthèse.

Les différents composés de départ sont connus ou peuvent être préparés par des procédés connus dont certains seront explicités plus précisément dans les exemples qui suivent.

Pour ce qui concerne les différentes sulfones préparées, leurs utilisations dans la synthèse de composés importants en chimie sont connues ou décrites dans l'article général mentionné précédemment.

Exemple 1

Préparation du phénylsulfonyl-3 butène-1
et du phénylsulfonyl-1 butène-2

On place dans un tube scellé 20 mg (0,1 mmole) de bis-$\pi$-allylchloropalladium préparé par le procédé de M. Sakakibara, Y. Takahashi, S. Sakai et Y. Ishii, Chem. Comm., (1969) 396, 82 mg (0,5 mmole) de phénylsulfinate de sodium, 52 mg (0,2 mmole) de triphénylphosphine, 1,41 g (10 mmoles) d'acide sulfinique, 10 ml de THF et 3,5 ml (40 mmoles) de butadiène et on laisse 65 heures à 20° C. Après ouverture, le solvant et le butadiène sont évaporés, le produit brut lavé avec une solution saturée de bicarbonate de sodium et extrait à l'éther.

Après séchage et évaporation de l'éther on obtient 2,0 g (9,5 mmoles) (95%) de mélange de sulfone

phénylsulfonyl-3 butène-1 et phénylsulfonyl-1 butène-2 dans la proportion respective de 80 pour 20 (par dosage CPV et comparaison du spectre RMN du brut avec celui d'un mélange des deux sulfones authentiques).

## Exemple 2

### Préparation du p-toluènesulfonyl-3 butène-1 et du p-toluènesulfonyl-1 butène-2

Dans un tube scellé on met 60 mg (0,3 mmole) de bis-$\pi$-allylchloropalladium, 266 mg (1,5 mmole) de p-toluènesulfinate de sodium, 396 mg (1,5 mmole) de triphénylphosphine et 4,7 g (30 mmoles) d'acide p-toluènesulfinique puis 15 ml de THF anhydre. Après avoir refroidi l'ensemble à −80° C on ajoute 5 ml (40 mmoles) de butadiène liquide.

Après 18 heures à 30° C, on récupère, après évaporation des solvants, lavage avec une solution saturée de bicarbonate de sodium et extraction à l'éther, 1,75 g de brut contenant le palladium, la triphénylphosphine et les sulfones.

Bilan établi par CPV et RMN:

| | |
|---|---|
| p-toluènesulfonyl-3 butène-1 | 30% |
| p-toluènesulfonyl-1 butène-2 (E) | 12%. |

Par cristallisation dans l'éther on sépare le p-toluènesulfonyl-3 butène-1 pur: 0,82 g (15% calculé sur l'acide sulfinique de départ).

| | |
|---|---|
| F = | 70° C (éther) |
| IR: | $cm^{-1}$ 1120, 1280. |
| RMN 80 MHz: | 1,43 (d, 7, 3H); 1,46 (s, 3H); 3,7 (m, 1H); 5 à 6,2 (m, 3H); centré à 7,6 (q, AA'BB', 4H). |
| Masse: | 210. |

p-toluènesulfonyl-3 butène-1 et p-toluènesulfonyl-1 butène-2 décrits par P. Bickart, F. W. Carson, J. Jacobus, E. G. Miller et K. Mislow J. Amer. Chem. Soc., (1968) 4869.

## Exemple 3

### Isomérisation du p-toluènesulfonyl-3 butène-1

On place dans un ballon 210 mg (1 mmole) de p-toluènesulfonyl-3 butène-1, 178 mg (1 mmole) de p-toluènesulfinate de sodium, 9 mg (0,5 mmole) d'eau et 2 ml de THF. On porte le tout à 60° C et on suit l'évolution par CPV:

- après 30 minutes on a 70% de sulfone primaire et 30% de secondaire,
- après 2 heures ou 7 heures on a 95% de sulfone primaire et 5% de secondaire.

Au bout de 7 heures on ajoute 50 ml d'éther et on filtre le p-toluènesulfinate. On récupère après évaporation des solvants 202 mg (96%) de sulfones qui sont:

| | |
|---|---|
| — p-toluènesulfonyl-1 butène-2 (E): | 64% |
| — p-toluènesulfonyl-1 butène-2 (Z): | 27% |
| — p-toluènesulfonyl-3 butène 1: | 5% |

Ces proportions sont établies par RMN d'après les données de P. Bickart, F. W. Carson, J. Jacobus, E. G. Miller et K. Mislow, J. Amer. Chem. Soc. (1968)4869, et le spectre du produit authentique.

## Exemple 4

### Préparation du phénylsulfonyl-2 méthyl-2 butène-3

Dans un tube qui sera ensuite scellé sous vide, on place 20 mg de bis-$\pi$-allylchloropalladium (0,1 mmole de Pd), 82 mg (0,5 mmole) de phénylsulfinate de sodium, 52 mg (0,2 mmole) de triphénylphosphine, 1,41 g (10 mmoles) d'acide phénylsulfinique puis 20 ml de tétrahydrofuranne et 15 ml ($\simeq$ 150 mmoles) d'isoprène. Après 72 heures à 20° C, on évapore le solvant et on obtient 2,25 g de brut.

Une analyse CPV du brut montre que l'on a un seul produit (SE 30; 2,5%; 3 m; Anachrom SD 70−80

mesh; 190° C).

Une chromatographie sur silice (Merck (60)® 70 à 230 mesh; ASTM) avec le chlorure de méthylène comme éluant donne 2 g (96%) de sulfone pure.

| | |
|---|---|
| Masse: | 210, 143, 77, 69. |
| Analyse: | $C_{11}H_{14}O_2S$. |
| RMN 80 MHz: | 143 (s, 6H), multiplet vinylique: (4,93, 5,22, 5,37 (2H)), (5,83, 6,01, 6,12, 6,3 (1H)), 7,45 à 8,05 (5H). |
| IR: | $cm^{-1}$ 1155, 1290. |

## Exemple 5

### Préparation du p-toluènesulfonyl-2 méthyl-2 butène-3

Dans un ballon préalablement placé sous argon, on place 40 mg (0,2 mmole de Pd) de bis-$\pi$-allylchloropalladium, 89 mg (0,5 mmole) de p-toluènesulfinate de sodium, 52 mg (0,2 mmole) de triphénylphosphine, 1,56 g (10 mmoles) d'acide p-toluènesulfinique puis 20 ml de tétrahydrofuranne et 2 ml (20 mmoles) d'isoprène. On laisse 18 heures à 45° C sous argon et, après évaporation du solvant et de l'isoprène, on isole 2,3 g de brut.

L'analyse par CPV indique la présence de sulfones dans les proportions suivantes:

- p-toluènesulfonyl-2 méthyl-2 butène-3: 93%
- p-toluènesulfonyl-1 méthyl-3 butène-2: 5%
- p-tolylallylsulfone: 2%.

Par chromatographie sur silice (Merck (60)®, 70 à 230 mesh, AST) avec le mélange (chlorure de méthylène 45/cyclohexane 45/éthyle 5) on isole 2,05 g (92%) de sulfone p-toluènesulfonyl-2 méthyl-2 butène-3 qui est ensuite recristallisée dans le mélange éther 50/pentane 50.

| | |
|---|---|
| F: | 51 – 52° C. |
| Analyse: | $C_{12}H_{16}O_2S$. |
| Masse: | 224. |
| RMN 80 MHz: | 1,375 (s, 6H), 2,38 (s, 3H) 4,90 à 5,40 (m, 2H), 6,0 (m, J = 10, J' = 17, 1H), 7,30 (d, 2H), 7,70 (d, 2H). |
| RMN$^{13}$C: | 20,8 ($2CH_3$), 21,6 ($CH_3$), 64,5 (C), 118,4 ($CH_2$), 128,7 (2CH), 130,2 (2CH), 132,0 (C), 136,4 (CH), 144,1 (C). |
| CPV: | 190° C, 7 mn. |

## Exemple 6

### Préparation du p-toluènesulfonyl-2 méthyl-2 butène-3

Dans un ballon sous argon on place 40 mg (0,2 mmole de Pd) de bis-$\pi$-allylchloro palladium, 1,78 g (10 mmoles) de p-toluènesulfinate de sodium, 260 mg (1 mmole) de triphénylphosphine, 10 ml de THF, 0,70 g (10 mmoles) d'acide acetique et 2 ml (20 mmoles) d'isoprène. On laisse 18 heures à 25° C sous argon puis on évapore le solvant et l'isoprène, on lave le brut avec une solution saturée de bicarbonate de sodium et on extrait à l'éther. Après séchage sur sulfate de magnésium et évaporation de l'éther on obtient 1,255 g de brut (41% de sulfones obtenues) qui contient aussi de la triphénylphosphine et des complexes colorés (brun rouge) du palladium. On dissout dans 20 ml de cyclohexane et on fait passer un courant d'air (100 ml/mn) pendant 30 minutes. Puis on filtre sur 10 g de silice pour plaques (Kieseigel 60 PF® 254 Merck) et on élue les sulfones de la silice par 50 ml d'éther, ce qui donne 0,820 g (36%) de sulfones après évaporation des solvants.

Les proportions respectives des sulfones sont obtenues par CPV:

- p-toluènesulfonyl-2 méthyl-2 butène-3: 92%
- p-toluènesulfonyl-1 méthyl-3 butène-2: 6%
- allyl p-tolylsulfone: 2%

Après recristallisation dans le mélange pentane/éther, on obtient 0,620 g (28%) de sulfone p-toluènesulfonyl-2 méthyl-2 butène-3 analogue en CPV, point de fusion et RMN à un échantillon authentique fabriqué d'après la technique de D. Savoia, C. Trombini et A. Umani-Ronchi, J. C. S. Perkin I, (1977) 123.

Exemple 7

Isomérisation de p-toluènesulfonyl-2 méthyl-2 butène-3

On chauffe à 60° C dans le tétrahydrofuranne (2 ml) de la sulfone phenylsulfonyl-2 méthyl-2 butène-3 (224 mg, 1 mmole) avec du p-toluènesulfinate de sodium (178 mg, 1 mmole) en suspension et de l'eau (9 mg, 0,5 mmole).

On suit par CPV l'apparition de la sulfone p-tolylsulfonyl-1 méthyl-3 butène-2 (SE 30, 2,5%, 3 m, sur Anachrom SD 70—80 mesh, 190° C) (10 mn). Au bout de 2,5 heures il s'en forme 50%, au bout de 8,5 heures 98%. On évapore alors le THF et on lave les solides à l'éther. Par évaporation de l'éther on isole 0,220 mg (98%) de sulfone p-tolylsulfonyl-1 méthyl-3 butène-2. La RMN montre que l'on a p-tolylsulfonyl-1 méthyl-3 butène-2 (spectre analogue à celui de l'authentique p-tolylsulfonyl-1 méthyl-3 butène-2 et à celui décrit par F. G. Bordwell et T. Mecca, J. Amer. Chem. Soc., (1972) 5829 et F. G. Bordwell et R. J. Kern, J. Amer. Chem. Soc., (1955) 1141), ainsi que la CPV et le point de fusion, F=80—81° C.

Exemple 8

Préparation du p-toluènesulfonyl-3 diméthyl-3,7 octadiène-1,6 (p-tolyllinalylsulfone)

Dans un ballon sous argon on met: 520 mg (3,5 mmoles) de p-toluènesulfinate de sodium, 9,2 g (59 mmoles) d'acide p-toluènesulfinique, 300 mg (1,9 mmole de Pd) de bis-$\pi$-allylchloro palladium, 1,9 g (7,5 mmoles) de triphénylphosphine (90 mmoles), 15 ml de myrcène et 60 ml de THF anhydre, puis on chauffe 18 heures à 60° C. On évapore le solvant, on lave le brut avec une solution saturée de bicarbonate de sodium et on extrait au chlorure de méthylène.

Une chromatographie sur colonne de silice (Merck (60)®, 70 à 230 mesh) éluée avec le mélange chlorure de méthylène 48/cyclohexane 48/acétate d'éthyle 4 nous donne (87%) 15 g de p-toluènesulfonyl-3 diméthyl-3,7 octadiène-1,6 (p-tolyllinalylsulfone) et (5,8%) 1 g de p-toluènesulfonyl-1 diméthyl-3,7 octadiène-2,6 (E).

p-toluènesulfonyl-3 diméthyl-3,7 octadiène-1,6 (p-tolyllinalylsulfone):

| | |
|---|---|
| F= | 70° C (pentane/éther) |
| CPV: | 210° C, 15 mn |
| RMN 250 MHz: | 1,38 (s, 3H), 1,57 (s, 3H), 1,67 (s, 3H) 1,92 (m, 4H), 2,44 (s, 3H), 5,1 (m, 1H), 5,1 (d, 17,5, 1H), 5,36 (d, 10,5, 1H), 5,9 à 6 (c, 10,5, 17,5, 1H), 7,30 (m, 2H), 7,68 (m, 2H). |
| Analyse: | $C_{17}H_{24}O_2S$ |
| Masse= | 293, 292, 157, 155... |
| RMN$^{13}$C: | 16,3 ($CH_3$), 17,6 ($CH_3$), 21,6 ($CH_3$), 22,5 ($CH_2$), 25,6 ($CH_3$), 32,9 ($CH_2$), 68,0 (C), 120,0 ($CH_2$), 122,8 (CH), 128,7 (2CH), 130,3 (2CH), 132 (2C), 135,0 (CH), 144,0 (C). |

p-toluènesulfonyl-1 diméthyl-3,7 octadiène-2,6 (géranyl et néryl sulfones par isomérisation de la linalylsulfone):

identifié à un échantillon authentique.

Exemple 9

Préparation de p-toluènesulfonyl-3 diméthyl-3,7 octadiène-1,6 (p-tolyllinalylsulfone)

Dans un ballon sous argon on met 1,78 g (10 mmoles) de p-toluènesulfinate de sodium, 260 mg (1 mmole) de triphénylphosphine, 40 mg (2 mmoles de Pd) de bis-$\pi$-allylchloro palladium, 0,60 g d'acide acétique (10 mmoles), 1,7 ml (10 mmoles) de myrcène er 10 ml de THF. Après 18 heures d'agitation à 25° C on évapore le solvant, on lave le brut avec une solution saturée de bicarbonate de sodium et on extrait à l'éther. Après séchage sur sulfate de magnésium, évaporation de l'éther et distillation du myrcène restant (tube à boule sous 11 mm Hg) (0,5 g=35%), le brut contient la triphénylphosphine et le palladium ainsi qu'un mélange de sulfones dont les taux d'obtention sont:

| | |
|---|---|
| — p-toluènesulfonyl-3 diméthyl-3,7 octadiène-1,6 (p-tolyllinalylsulfone): | 35%, |
| — p-toluènesulfonyl-1 diméthyl-3,7 octadiène-2,6 (E): | 6,7% |
| — p-toluènesulfonyl-1 diméthyl-3,7 octadiène-2,6 (Z): | 1,3%, |

calculés par CPV.

Ces sulfones ont été comparées aux données de la littérature et à celles obtenues précédemment en RMN proton, RMN $^{13}C$, CPV et températures de fusion.

Exemple 10

Préparation de p-toluènesulfonyl-1 diméthyl-3,7 octadiène-2,6
(géranyl et néryl sulfones par isomérisation de la linalylsulfone)

On met 0,292 g (1 mmole) de p-toluènesulfonyl-3 diméthyl-3,7 octadiène-1,6 (p-tolyllinalylsulfone), 0,178 g (1 mmole) de p-toluènesulfinate de sodium, 2 ml de THF et 9 mg (0,5 mmole) d'eau dans un ballon. On chauffe à 60° C pendant 40 heures puis on ajoute 50 ml d'éther et on filtre. Après évaporation du filtrat on obtient 0,290 g (99%) de sulfones p-toluènesulfonyl-1 diméthyl-3,7 octadiène-2,6 en proportions Z/E = 20/80. Par chromatographie sur plaque de silice (Merck) éluée par le mélange $CH_2Cl_2$ 50/cyclohexane 50/acétate d'éthyle 5 on récupère quantitativement 58 mg (20%) de p-toluènesulfonyl-1 dimétjyl-3,7 octadiène-2,6 (Z) et 230 mg (79%) de p-toluènesulfonyl-1 diméthyl-3,7 octadiène-2,6 (E).

— p-toluènesulfonyl-1 diméthyl-3,7 octadiène-2,6 (Z): huile

| | |
|---|---|
| analyse: | $C_{17}H_{24}O_2S$. |
| CPV: | 210° C, 16 mn. |
| Masse: | 292. |
| RMN 250 MHz: | 1,54 (s, 3H), 1,66 (s, 3H), 1,74 (s, 3H), massif, 1,75 à 2 (m, 4H), 2,44 (s, 3H), 3,78 (d, 8, 2H), 4,95 (t, 7, 1H), 5,20 (t, 8, 1H), 7,32 (m, 2H), 7,74 (m, 2H). |
| RMN $^{13}C$: | 17,8 ($CH_3$), 21,7 ($CH_3$), 23,7 ($CH_3$), 25,7 ($CH_3$), 26,2 ($CH_2$), 32,0 ($CH_2$), 56,1 ($CH_2$), 110,9 (CH), 132,2 (CH), 128,2 (2CH), 129,4 (2CH), 132,0 (C), 136,0 (C), 144,1 (C), 145,5 (C). |

— p-toluènesulfonyl-1 diméthyl-3,7 octadiène-2,6 (E): est recristallisée dans pentane/éther.

| | |
|---|---|
| F = | 44° C |
| CPV: | 210° C, 19 mn. |
| Masse: | 292. |
| RMN 80 MHz: | 1,36 (s, 3H), 1,61 (s, 3H), 1,70 (s, 3H), 1,95 à 2,15 (m, 4H), 2,45 (s, 3H), 3,81 (d, 8, 2H), 4,85 à 5,50 (m, 2H), 7,38 (m, 2H), 7,83 (m, 2H). |
| RMN $^{13}C$: | 16,3 ($CH_3$), 17,8 ($CH_3$), 21,7 ($CH_3$), 25,7 ($CH_3$), 26,3 ($CH_2$), 39,7 ($CH_2$), 56,2 ($CH_2$), 110,3 (CH), 123,3 (CH), 128,3 (2CH), 129,2 (2CH), 131,7 (C), 135,7 (C), 144,1 (C), 145,7 (C). |

Attribution des structures (E) et (Z) pour p-toluènesulfonyl-1 diméthyl-3,7octadiène-2,6 d'après L. grombie, R. V. M. Campbell, D. A. R. Finlley, R. W. King, G. Pattenden et D. A. Whiting, J. C. S. Perkin I, (1975) pp. 897 à 915 donnant les RMN proton et $^{13}C$ des géranyl et néryl phénylsulfones, de même que M. Julia et D. Uguen, Bull. Soc. Chim. France (1976) 513.

Exemple 11

Préparation de p-toluènesulfonyl-2 pentène-3, de p-toluènesulfonyl-1 pentène-2
et de p-toluènesulfonyl-3 pentène-1

Dans un ballon sous argon on place 40 mg (0,2 mmole de Pd) de bis-$\pi$allylchloro palladium, (0,5 mmole), 89 mg de p-toluènesulfinate de sodium, 52 mg (0,2 mmole) de triphénylphosphine, 1,56 g (10 mmoles) d'acide p-toluènesulfinique, 2 ml de pipérylène (20 mmoles) et 20 ml de THF. On chauffe le tout 18 heures à 40° C. On évapore le solvant puis on lave le brut avec une solution saturée de bicarbonate de sodium et on extrait au chlorure de méthylène. On récupère 2,30 g de mélange des sulfones p-toluènesulfonyl-2-pentène-3, p-toluènesulfonyl-1 pentène-2 et p-toluènesulfonyl-3 pentène-1 avec la triphénylphosphine et le palladium. On dose les sulfones isomères:

| | |
|---|---|
| — p-toluènesulfonyl-2 pentène-3 (E) | (70%) |
| — p-toluènesulfonyl-1 pentène-2 (E) | (10%) |
| — p-toluènesulfonyl-3 pentène-1 | (15%) |
| — p-tolylallylsulfone | (2%) |

par CPV et par RMN 250 MHz du brut en comparaison avec les échantillons authentiques.

**Revendications**

1. Procédé de préparation de sulfones ou d'un mélange de sulfones de formule I:

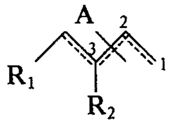

(I)

dans laquelle la ligne pointillée représente une double liaison en position 1, 2 ou 3, A représente un radical alkylsulfonyle, alkenylsulfonyle ou arylsulfonyle, placé en $\alpha$ de ladite double liasidon, $R_1$ et $R_2$ représentent l'hydrogène, un radical alkyle, alkényle ou alkynyle non substitués ou bien substitués par une ou plusieurs fonctions hydroxy ou oxo et/ou par des radicaux cycloalkyles ou cycloalkényles qui peuvent eux-mêmes être substitués par un ou plusieurs radicaux méthyles, caractérisé par la sulfonation en présence d'un catalyseur au palladium d'un composé diénique de formule II:

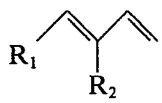

(II)

dans laquelle $R_1$ et $R_2$ ont les significations données précédemment, à l'aide d'au moins un agent introduisant le radical sulfonyle A, choisi parmi:

- les acides sulfiniques,
- les sulfinates en présence d'un acide, dans un solvant, séparation des sulfones et isomérisation de certaines d'entre elles si nécessaire.

2. Procédé selon la revendication 1, caractérisé en ce que le radical A est un radical arylsulfonyle.
3. Procédé selon la revendication 2, caractérisé en ce que l'agent intriduisant le radical arylsulfonyle A est un mélange d'acide arylsulfinique et d'un arylsulfinate correspondant.
4. Procédé selon la revendication 2, caractérisé en ce que l'agent introduisant le radical arylsulfonyl A est un acide arylsulfinique.
5. Procédé selon la revendication 2, caractérisé en ce que l'agent introduisant le radical arylsulfonyle A est un arylsulfinate en présence d'acide acétique ou formique.
6. Procédé selo l'une des revendications 1 à 5, caractérisé en ce que le catalyseur au palladium erst sinstutué d'un sel ou d'un complexe du palladium et d'une phosphine.
7. Procédé selon la revendication 6, caractérisé en ce que le complexe du palladium erst choisi parmi:

- und dérivé $\pi$-allyle palladié, et
- le tétrakis-triphénylphosphine palladium.

8. Procédé selon l'une des revendications 6 et 7, caractérisé en ce que la phosphine est la triphényl-phosphine.
9. Procédé selon l'une des revendications 1 à 8, caractérisé en ce que le solvant est le tétrahydrofuranne.
10. Procédé selon l'une des revendications 1 à 9 caractérisé en ce que le composé diénique de formule II est choisi parmi:

- le butadiène,
- le pipérylène,
- le myrcène,
- l'isoprène.

11. Procédé selon l'une des revendications 1 à 10, caractérisé en ce que l'on isomérise une sulfone de formule Ia:

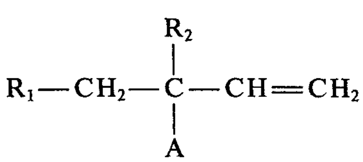

(Ia)

en une sulfone de formule Ib:

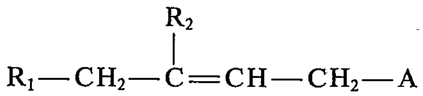

(Ib)

par action d'un sulfinate en présence d'eau et d'un solvant organique.

12. Procédé selon la revendication 11, caractérisé en ce que le sulfinate utilisé pour l'isomérisation est un sulfinate correspondant au radical A des sulfones Ia et iB, et en ce que le solvant organique est le tétrahydrofuranne.

13. Procédé selon l'une des revendications 11 et 12, caractérisé en ce que l'isomérisation est conduite dans le mélange de sulfonation.

**Patentansprüche**

1. Verfahren zur Herstellung von Sulfonen oder einer Sulfonmischung nach der Formel I:

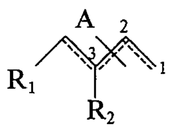

(I)

in welcher die punktierte Linie eine doppelte Verbindung in Stellung 1, 2 oder 3 darstellt, A ein Alkylsulfonyl-, Alkenylsulfonyl- oder Arylsulfonyl-Radikal in $\alpha$-Stellung zur genannten Doppelverbindung angebracht, $R_1$ und $R_2$ Wasserstoff, ein Alkyl-, Alkenyl- oder Alkynyl-Radikal, die nicht substituiert oder aber durch ein oder mehrere Hydroxy- oder Oxo-Funktionen und/oder durch ein Cycloalkyl oder Cycloalkenyl-Radikale substituiert sein können, die selbst durch ein oder mehrere Methylradikale substituiert sein können, gekennzeichnet durch die Sulfonierung einer Dien-Verbindung der Formel II:

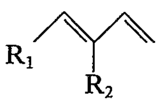

(II)

in welcher $R_1$ und $R_2$ die vorhin angegebene Bedeutung haben, in Gegenwart eines Palladiumkatalysators, mit Hilfe mindestens eines das Sulfonyl-Radikal A einführenden Mittels, gewählt aus:

- Sulfinsäuren,
- Sulfinaten in Gegenwart einer Säure,

und in einem Lösungsmittel, Trennung der Sulfone und Isomerisation bestimmter unter ihnen, wenn notwendig.

2. Verfahren nach Anspruch 1, dadurch gekennzeichnet, daß A ein Arylsulfonyl-Radikal ist.

3. Verfahren nach Anspruch 2, dadurch gekennzeichnet, daß das Arylsulfonyl-Radikal A einführende Mittel eine Mischung einer Arylsulfinsäure und dem entsprechenden Arylsulfinat ist.

4. Verfahren nach Anspruch 2, dadurch daß das das Arylsulfonyl-Radikal A einführende Mittel eine Arylsulfinsäure ist.

5. Verfahren nach Anspruch 2, dadurch gekennzeichnet, daß das das Arylsulfonyl-Radikal A einführende Mittel ein Arylsulfinat in Gegenwart von Essig- oder Ameisensäure ist.

6. Verfahren nach einem der Ansprüche 1 bis 5, dadurch gekennzeichnet, daß der Palladiumkatalysator aus einem Salz oder einem Palladiumkomplex und einem Phosphin gebildet wird.

7. Verfahren nach Anspruch 6, dadurch gekennzeichnet, daß der Palladiumkomplex unter:

- einem $\pi$-Allyl-Palladium-Derivat und
- dem tetrakis-Triphenylphosphin-Palladium

gewählt wird.

8. Verfahren nach einem der Ansprüche 6 und 7, dadurch gekennzeichnet, daß das Phosphin das Triphenylphosphin ist.

9. Verfahren nach einem der Ansprüche 1 bis 8, dadurch gekennzeichnet, daß das Lösungsmittel Tetrahydrofuran ist.

10. Verfahren nach einem der Ansprüche 1 bis 9, dadurch gekennzeichnet, daß die Dien-Verbindung der Formel II unter:

— Butadien,
— Piperylen,
— Myrcen,
— Isopren gewählt wird.

11. Verfahren nach einem der Ansprüche 1 bis 10, dadurch gekennzeichnet, daß man ein Sulfon der Formel Ia:

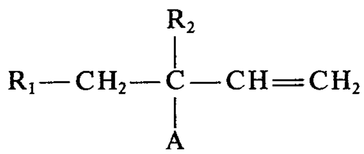

(Ia)

in ein Sulfon der Formel Ib isomerisiert,

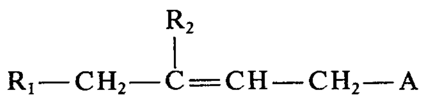

(Ib)

durch die Wirkung eines Sulfinates in Gegenwart von Wasser und einem organischen Lösungsmittel.

12. Verfahren nach Anspruch 11, dadurch gekennzeichnet, daß das für die Isomerisation verwendete Sulfinat ein dem Radikal A der Sulfone Ia und Ib entsprechendes Sulfinat ist und daß das organische Lösungsmittel das Tetrahydrofuran ist.

13. Verfahren nach einem der Ansprüche 11 und 12, dadurch gekennzeichnet, daß die Isomerisation in der Sulfoniermischung durchgeführt wird.

**Claims**

1. A process for the preparation of sulphones or a mixture of sulphones corresponding to formula I:

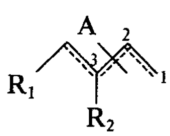

(I)

wherein
the dashed line represents a double bond in the 1, 2 oder 3 position,
A represents an alkylsulphonyl, alkenylsulphonyl or arylsulphonyl radical, in the $\alpha$ position relative to said double bond, and
$R_1$ and $R_2$ represent hydrogen, an alkyl, alkenyl or alkynyl radical which are not substituted or are substituted by one or more hydroxy or oxo functions and/or by cycloalkyl or cycloalkenyl radicals which may themselces be substituted by one or more methyl radicals, charaterised by sulphonation, in the presence of a palladium catalyst, of a dien diene compound corresponding to formula II:

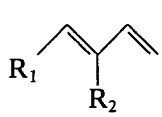

(II)

wherein
$R_1$ and $R_2$ are as defined above,
using at least one agent introducing the sulphonyl radical A, selected from:
sulphinic acids,
sulphinates in the presence of an acid,
in a solvent, separating the sulphones and isomerising some of them, if necessary.

2. A process according to claim 1, characeterised in that the radical A ist an arylsulphonyl radical.

3. A process according to claim 2, characterised in that the agent introducing the arylsulphonyl radical A is a mixture of arylsulphinic acid and a corresponding arylsulphinate.

4. A process according to claim 2, characterised in that the agent introducing the arylsulphonyl radical A is an arylsulphinic acid.

5. A process according to claim 2, charaterised in that the agent introducing the arylsulphonyl radical A ist an arylsulphinate in the presence of acetic or formic acid.

6. A process according to one of claims 1 to 5, characterised in that the palladium catalyst consists of

a salt or a complex of palladium and a phosphine.

7. A process according to claim 6, charaterised in that the palladium complex is selected form among the following:
a $\pi$-allyl palladinized derivative, and
palladium tetrakis-triphenylphosphine.

8. A process according to one of claims 6 and 7, characeterised in that the phosphine is triphenylphosphine.

9. A process according to one of claims 1 to 8, characterised in that the solvent is tetrahydrofuran.

10. A process according to one of claims 1 to 9, characterised in that the dien diene compound corresponding to forumla II is selected from among the following:

butadiene,
piperylene,
myrcene, and
isoprene.

11. A process according to one of claims 1 to 10, characterised in that a sulphone corresponding to formular Ia:

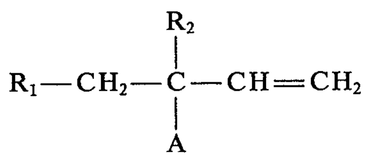

(Ia)

is isomerised into a sulphone corresponding to formula Ib:

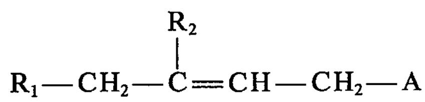

(Ib)

by the action of a sulphinate in the presence of water and an organic solvent.

12. A process according to claim 11, characterised in that the sulphinate which is used for isomerisation is a sulphinate which is used for isomerisation is a sulphinate corresponding to the radical A of the sulphones Ia and Ib, and the organic solvent is tetrahydrofuran.

13. A process according to claims 11 and 12, characterised in that isomerisation is carried out in the sulphonation mixture.